(19) Europäisches Patentamt
European Patent Office
Office européen des brevets

(11) Publication number: **0 070 016**
**A1**

# (12) EUROPEAN PATENT APPLICATION

(21) Application number: 82106177.7

(22) Date of filing: 12.07.82

(51) Int. Cl.$^3$: **A 23 K 1/16**

(30) Priority: 13.07.81 US 282843

(43) Date of publication of application: 19.01.83 Bulletin 83/3

(84) Designated Contracting States: BE CH DE FR GB IT LI NL SE

(71) Applicant: THE UNIVERSITY OF UTAH
201 Biology Building
Salt Lake City Utah 84112(US)

(72) Inventor: Gardner, Pete Delos
2200 S. 2300E
Salt Lake City Utah 84109(US)

(72) Inventor: Negus, Norman Curtiss
1960 Alla Panna Way
Sandy Utah 84029(US)

(72) Inventor: Negus, Patricia Jane
1960 Alla Panna Way
Sandy Utah 84029(US)

(72) Inventor: Sanders, Edward Henry
130 South 500 East Apt, 203
Salt Lake City Utah 84115(US)

(74) Representative: Casalonga, Axel et al,
BUREAU D.A. CASALONGA OFFICE JOSSE & PETIT
Baaderstrasse 12-14
D-8000 München 5(DE)

## (54) Method and composition for increasing reproduction in mammalian and avian species.

(57) Reproductive performance in mammalian and avian species is stimulated by administering an effective amount of one or more compounds of any of Formulas I, II or III, typically at the rate of 0.01 to 2 mg/kg/day:

(I) a compound of the formula:

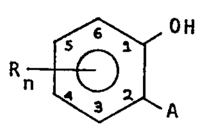

wherein

R represents $C_1$-$C_4$ alkoxy, with the proviso that R is in the 4 or 5 ring position,

n represents the integers 0, 1 or 2, and

A represents $-OH$, $-NH_2$ or

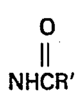

where R' represents $C_1$-$C_4$ alkyl,

or physiologically acceptable salts thereof;

(II) a compound of the formula:

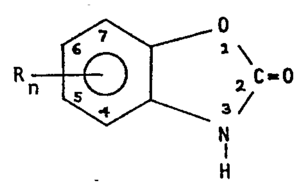

wherein

R represents $C_1$-$C_4$ alkoxy, with the proviso that R is in the 5 or 6 ring position, and

n represents one of the integers 0, 1 or 2;

and

(III) a compound of the formula:

./...

EP 0 070 016 A1

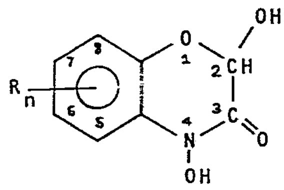

wherein

R represents $C_1$-$C_4$ alkoxy, with the proviso that R is in the 6 or 7 ring position, and

n represents one of the integers 0, 1 or 2;

or physiologically acceptable salts thereof.

The active compounds are incorporated in novel feed concentrates and feed compositions or parenteral dosage forms or implants utilized according to the recited method.

METHOD AND COMPOSITION FOR INCREASING REPRODUCTION IN MAMMALIAN AND AVIAN SPECIES

The present invention relates to a method of increasing the reproductive performance in mammalian and avian species.

According to Science, 196, pp. 1230-1231, (1977), fresh green wheatgrass was fed to a nonbreeding winter population of *Microtus montanus* (a species of rodent) for 3 weeks. All females receiving the green wheatgrass were pregnant at the end of the feeding period while animals in the control, which were fed no green wheatgrass, remained in a nonbreeding condition. This data suggest that *Microtus montanus* cues reproductive effort from chemical signals in the plant food resources.

Negus et al., Journal of Mammalogy, Vol. 47, No. 4, December 2, 1966, pp. 596-601, observed the reproductive responses of *Microtus montanus* to plants and plant extracts in the diet. Negus et al. suggest that hormone-like substances in green plants may influence reproduction in natural populations of *Microtus montanus*.

It is believed that heretofore, the active ingredient or factor present in green plants that increases reproduction in Microtus montanus has not been identified.

It has been unexpectedly found that the activity of green plants for the stimulation of reproduction in Microtus montanus is due to 2,4-dihydroxy-7-methoxy-1,4-(2H)-benzoxazine-3-one and its enzymatically derived injury compound 6-methoxy-2-benzoxazolinone (6-MBOA).

The present invention relates to a method of stimulating reproduction in mammalian and avian species, said method comprising administering to said mammalian and avian species an effective reproductive stimulating amount of one or more active compounds of Formulas I, II or III:

(I) a compound according to the formula

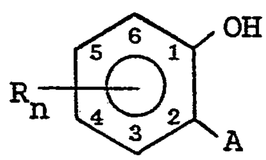

wherein

R represents $C_1$-$C_4$ alkoxy, with the proviso that R is in the 4 or 5 ring position,

n represents one of the integers 0, 1 or 2, and

A represents -OH, $-NH_2$ or $NH\overset{O}{\overset{\|}{C}}R'$ where R' represents $C_1$-$C_4$ alkyl,

or a physiologically acceptable salt thereof;

(II) a compound according to the formula

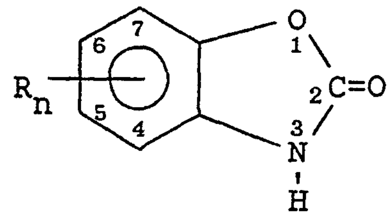

wherein

R represents $C_1$-$C_4$ alkoxy, with the proviso that R is in the 5 or 6 ring position, and

n represents one of the integers 0, 1 or 2;

and

(III) a compound according to the formula

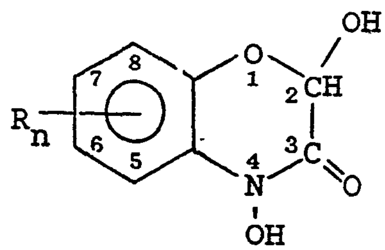

wherein

R represents $C_1$-$C_4$ alkoxy, with the proviso that R is in the 6 or 7 ring position, and

n represents one of the integers 0, 1 or 2,

or a physiologically acceptable salt thereof.

Additionally, the present invention relates to a reproductive stimulating composition comprising an active compound of Formula (I), (II) or (III) in admixture with an adjuvant therefor, said active compound being present in an amount of at least 0.1 parts per million by weight. Said compositions are typically animal feeds.

The term "stimulating reproductive performance" refers to the effects exhibited by an animal in response to an effective reproductive amount of any one or more of the compounds of Formula I, II or III. Said effects include, for example, any one or more of the following: increased litter size, increased frequency of post partum mating, early onset of sexual maturation, return to breeding activity of non-breeding winter animals, increased egg laying, increased length of effective reproductive life and increased number of fertile eggs laid. Both males and females exhibit reproductive effects when fed effective amounts of any one or more of the active compounds of Formula I, II or III.

An important objective of this invention is to increase the production of agriculturally important animals, such as, cattle, horses, rabbits, sheep, swine and poultry. Fulfilling this objective will increase the available food supplies while at the same time making it more economical for commercial growers to raise these agriculturally important animals. A preferred group of animals, in which an increase in reproduction is desirous, is the avian species, such as, for example, chickens, turkeys, ducks, quail and geese.

The compounds of Formula I, II and III can be administered to animals orally by dosage forms, such as, in admixture with feed, feed concentrates or supplements and additionally in the form of boluses, capsules, tablets, suspensions or solutions containing said compounds. The compounds can also be administered parenterally, such as, for example, intramuscularly or intravenously, or by way of an implant which slowly releases the compound into the tissue or blood stream of the animal in an effective reproductive amount.

The effective reproductive amount of the present compounds exists as a range of from 0.01 to 2 milligrams per kilogram of body weight of animal per day. This effective range can vary depending on many factors, such as, the size of the animal, the species of the animal, the age of the animal, the active compound used or the route of administration of the active compound. The optimum range of an effective amount, based on the above-mentioned variables, can be found using conventionally known techniques, i.e., dose titration determinations.

An effective reproductive amount of the present compound can be conveniently administered substantially daily prior to the onset of maturation of an animal and throughout the time that an animal is capable of reproducing. "Substantially daily" administration of the active compounds described herein is meant to encompass dosage schedules, such as, for example, every other day administration and administration 5 or 6 days in a 7 day period, all of which are within the scope of the present invention.

Suitable representative compounds of Formula I, II and III that stimulate reproductive performance in mammals and birds include the following compounds, and, in the case of each such compound carrying an OH function, the physiologically acceptable salts thereof:

2-amino-5-methoxyphenol,
6-methoxy-2-benzoxazolinone (6-MBOA),
2,4-dihydroxy-7-methoxy-1,4-(2H)-
-benzoxazin-3-one,
2-hydroxy-4-methoxyacetanilide,
2-hydroxy-4-ethoxyacetanilide,
5-methoxy-2-benzoxazolinone,
2-amino-4-methoxyphenol, and
2-hydroxy-5-methoxyacetanilide.

The present compounds are conveniently incorporated in a feed composition in an appropriate amount to achieve the desired daily dosage. This amount will vary depending upon the amount of feed composition consumed daily by the animal. For example, one or more of the present compounds are conveniently incorporated in a chicken feed composition from generally 0.1 to 50 grams per ton (907.18 kg) of feed (0.00001% to 0.0052% by weight)(0.1-52 ppm). The present compounds may also be incorporated in a mineral, protein or energy-type feed additive supplement or water supply in an appropriate amount to provide an effective reproductive daily dosage.

For commercial use, it is convenient to provide a feed additive premix, mineral supplement or concentrate containing one or more of the present compounds in a proportion such that a predetermined quantity of the premix is to be added per ton of complete feed, for example, from 0.1 (.0453 kg) to 1,000 pounds (453.59 kg) contains from 0.1 to 50 grams of the present compounds. The feed additive premix or concentrate comprises one or more of the present compounds and a carrier such as soybean meal or ground corn or other edible feed grade material, mineral mixtures or innocuous diluent, such as, alcohols, glycols or molasses, suitable for the animal at hand. A concentrate may contain from 0.001 to 99 percent by weight of one or more of the present compounds in intimate admixture with an adjuvant therefor.

The present compounds, when administered parenterally, are preferably dissolved in sterile

distilled water, propylene glycol or other physiologically acceptable liquid media and compounded in accordance with the known pharmaceutical art.

Representative of animals which exhibit an increased reproductive performance when administered an effective reproductive amount of one or more of the present compounds include commercial animals, such as, cattle, horses, sheep, swine and poultry and laboratory animals, such as, rabbits, rodents, including rats, mice and voles. While rodents are not ordinarily considered meat-bearing animals, many rodents are now being raised for toxicology and similar industrial studies, and have been found to be a rather accurate indicator of the physiological effects of feeds and feed additives fed to larger warm-blooded animals.

In further embodiments, the method of the present invention contemplates treating or dosing an animal with novel compositions containing at least one of the present compounds as the active ingredient which also can be advantageously employed in combination with one or more additional additives such as coccidiostats, antibiotics, minerals, vitamins or growth promotants employed in animal husbandry.

The animal feeds most generally used in conjunction with this invention are composed of various grain and/or grain mixtures and/or roughage feeds such as hay, cotton seed hulls, rice hulls, silage, or other high fiber feedstuffs commonly fed to meat-, milk-, and/or wool-producing animals, especially in cattle or sheep feeds. The feeds for swine, poultry, and laboratory animals will consist primarily of various grain mixtures

plus the usual additaments such as bran meal, soybean meal, cotton seed meal, tankage or alfalfa meals suitable for monogastric animals.

Examples of carriers for premix or concentrate compositions are soybean meal, corn oil, ground corn, ground corn cobs, barley, wheat, mineral mixtures containing, e.g., vermiculite or diatomaceous earth, corn gluten meal, corn distillers' solubles, soy flour or other modestly priced edible ingredients. The active ingredient will be used in amounts to satisfy the criteria set forth above for balanced feeds. This premix or concentrate is then in turn mixed uniformly with the normal diet for the animal as desired by the grower or the feed mixer. The above mentioned grains, grain mixtures, roughage feeds, usual additaments, carriers and innocuous diluents constitute acceptable adjuvants for purposes of this invention.

The dosage of a preferred compound, 6-methoxy--2-benzoxazolinone (6-MBOA), in chickens, falls in the range of from 0.01 to 2 milligrams per kilogram of body weight per day (mg/kg/day), i.e., 0.1-50 grams/ton (907.18 kg) of feed, and more preferably, in the range of from 0.02 to 1.5 mg/kg/day. Representative of one method of administration of 6-MBOA to chickens, one gram of synthetically produced, substantially pure 6-MBOA is dissolved in 250 milliliters of a 50/50 methanol/ether solution and thoroughly blended with 5 lbs. (2.268 kgs) of corn meal. The methanol/ether solvent is allowed to evaporate and then the 5 lbs. (2.268 kgs) of corn meal containing the 6-MBOA is thoroughly blended into 995 lbs. (451.324 kgs) of finished chicken feed.

The following examples further illustrate the practice of the present invention. All percentages are by weight unless specified otherwise.

Example I

A complete developing ration to be fed to broiler breeder pullets and cockerels during the period from 10 to 20 weeks of age comprises:

| | |
|---|---|
| Crude Protein not less than | 11.0% |
| Crude fat not less than | 2.5% |
| Crude fiber not more than | 4.5% |

supplied by the following ingredients: Grain products, processed grain by-products, plant protein products, animal protein products, forage products, animal fat preserved with BHA (butylated hydroxyanisole), cane molasses, ethoxyquin (a preservative), methionine hydroxy analogue calcium, vitamin $B_{12}$ supplement, vitamin A supplement, calcium pantothenate, choline chloride, riboflavin supplement, D activated animal sterol, folic acid, menadione dimethylpyrimidinol bisulfite (source of vitamin K activity), niacin, defluorinated phosphate, magnesium sulfate, potassium sulfate, calcium carbonate, iodized salt, manganous oxide, copper sulfate, zinc oxide. Such a formulation is commercially available as Purina® Broiler Breeder Developer (G) from the Ralston Purina Co.

Example II

A complete developing ration to be fed to boiler breeder pullets and cockerels during the period

from 10 to 20 weeks of age which causes an incease in reproductive performance in said birds comprises:

| | |
|---|---|
| Ration of Example I | 1 ton (907.18 kg) |
| 6-MBOA | 0.1-5 gram per ton (907.18 kg) of ration |

The feed is prepared by dissolving the 6-MBOA in a 50/50 methanol/ether solution and thoroughly blending this solution into 5 pounds (2.268 kgs) of corn meal. The methanol/ether solvent is then allowed to evaporate. The 5 pounds (2.268 kgs) of corn meal containing the 6-MBOA is then thoroughly blended into 1995 pounds (904.917 kgs) of ration to make 1 ton of feed.

Example III

A fortified complete ration to be fed to broiler breeders from 20 weeks of age through the breeding period comprises:

| | |
|---|---|
| Crude protein not less than | 15.0% |
| Crude fat not less than | 3.0% |
| Crude fiber not more than | 4.5% |
| Calcium (Ca) not less than | 2.8% |
| Calcium (Ca) not more than | 3.8% |
| Phosphorus (P) not less than | 0.6% |
| Iodine (I) not less than | 0.0001% |
| Salt (NaCl) not less than | 0.3% |
| Salt (NaCl) not more than | 0.9% |

supplied by the following ingredients: Grain products, processed grain by-products, plant protein products,

animal protein products, dried whey fermentation solubles, forage products, cane molasses, methionine hydroxy analogue calcium, ethoxyquin (a preservative), animal fat preserved with BHA (butylated hydroxyanisole), vitamin $B_{12}$ supplement, choline chloride, calcium pantothenate, riboflavin supplement, vitamin A supplement, Vitamin E supplement, D activated animal sterol, folic acid, menadione dimethylpyrimidinol bisulfite (source of vitamin K activity), niacin, defluorinated phosphate, magnesium sulfate, potassium sulfate, calcium carbonate, iodized salt, sodium selenite, manganous oxide, copper sulfate, zinc oxide. Such a formulation is commercially availble as Purina® Broiler Breeder Eggena® (G) from the Ralston Purina Co.

Example IV

A fortified complete ration to be fed to broiler breeders from 20 weeks of age through the breeding period which causes an increase in reproductive performance in said birds comprises:

| | |
|---|---|
| Ration of Example III | 1 ton (907.18 kg) |
| 6-MBOA | 0.1-5 grams per ton (907.18 kg) of ration |

The feed is prepared by dissolving the 6-MBOA in a 50/50 methanol/ether solution and thoroughly blending this solution into 5 pounds (2.268 kgs) of corn meal. The methanol/ether solvent is then allowed to evaporate. The 5 pounds (2.268 kgs) of corn meal containing the 6-MBOA is then thoroughly blended into 1995 pounds (904.917 kgs) of ration to make 1 ton (907.18 kgs) of feed.

Example V

One hundred and ninety-two (192) virgin, 8 week old Microtus montanus were paired for breeding. Each pair was maintained separately in a fiberglass cage with a wire mesh top and a sawdust substrate with cotton batting for nesting material. Food and water were available ad lib. The animals were maintained at a photoperiod of 16 hours of light and 8 hours of dark per 24 hour period and a room temperature of 20°C. The 96 pairs of animals were divided into 4 treatment groups of 24 pairs each. Each group received a distinct dietary treatment consisting of a basal diet of Purina brand rabbit chow coated with varying amounts of synthetically produced, substantially pure 6-methoxybenzoxazolinone (6-MBOA). The 6-MBOA was dissolved in an ether/ethanol solvent (4 parts by weight ether to 1 part ethanol) and then mixed with the chow. The solvent was allowed to evaporate before feeding. The control group was fed the chow coated with the ether/ethanol solvent only. The different groups were fed 6-MBOA as follows:

| Group | Amount 6-MBOA |
|---|---|
| 1 | None (Control) |
| 2 | 0.1 μg/g of chow |
| 3 | 1.0 μg/g of chow |
| 4 | 10.0 μg/g of chow |

The mated pairs were checked regularly for births and the number of young. Offspring were sexed and weaned at 17 or 18 days of age. The pairs were maintained for 117 days of mated life after which the animals were sacrificed and the females were examined

for signs of pregnancy. M. montanus has a gestation period of 21 days and can mate in post-partum. Thus, 117 days was adequate time for the maximum production of 5 litters. The results were evaluated on the basis of average litter size, frequency of litters and number of young and are listed in Table I.

TABLE I

| | I (Control) | II (0.1 μg 6-MBOA/g Chow) | III (1 μg 6-MBOA/g Chow) | IV (10 μg 6-MBOA/g Chow) |
|---|---|---|---|---|
| Average Number of Litters | 3.4 | 3.6 | 3.8 | 3.9 |
| *Total Number of Young Born | 311 | 344 | 364 | 371 |
| Total Number of Young from ♀ Who Survived Whole Experiment | 302 | 319 | 347 | 332 |
| Average Number of Offspring/Female | 15.1 | 16.8 | 19.3 | 18.4 |
| Average Litter Size | 4.4 | 4.6 | 4.8 | 5.2 |

* Indicates that data includes litters from females who did not survive the whole experiment.

♀ Indicates female.

Example VI

One hundred and forty-four (144) Bobwhite quail, between 2 and 3 months old, were paired for breeding. Each pair was caged individually in a wire mesh cage and food and water were available ad lib. The birds were initially divided into two groups of 36 pairs each. All birds were kept in the same room. Group 1 received Purina brand startina gamebird chow which had been coated with an ether/ethanol solvent (4 parts by weight ether per part of ethanol) by mixing the solvent with the chow and allowing solvent to evaporate before feeding. Group 2 received the same chow as Group 1 except that the chow was coated with synthetically produced, substantially pure 6-MBOA in an amount of 1.04 μg 6-MBOA/g of chow by dissolving the 6-MBOA in a ether/ethanol (4:1) solvent. Both groups were maintained under a photoperiod of 16 hours of light and 8 hours of dark per 24 hour period for 5 weeks. During this time no eggs were produced. At the beginning of the sixth week, the photoperiod was reversed to 8 hours of light and 16 hours of dark and all animals in both groups received control chow only. These light and feed conditions were maintained for 6 1/2 weeks or until the middle of the 12th week of the experiment. In the middle of the 12th week the photoperiod was returned to 16 hours of light and 8 hours of darkness per day. Group 1 continued on control chow and Group 2 received 6-MBOA coated chow at a level of 10.0 μg/g of chow. Half way through the 21st week 18 of the 36 pairs in Group 2 had their diet changed to a lower dose of 6-MBOA, i.e., 1.0 μg 6-MBOA/g of chow, while the remaining animals in Group 2 continued on 10 μg 6-MBOA/ g of chow.

The birds were monitored daily for egg production. The eggs were identified as to the individuals which laid them and the eggs were weighed. Subsequently, the eggs were incubated and candled after 5-7 days of incubation to determine fertility. The data relating to eggs/female/week and percent egg fertility are indicated in Tables II, III and IV respectively.

## TABLE II

Number of Eggs Laid

| Week | Group 1 (Control) | | | Group 2 (10 µg 6-MBOA/g chow) | | |
|---|---|---|---|---|---|---|
| | #eggs/ week | # hens | #eggs/ hen/week | #eggs/ week | # hens | #eggs/ hen/week |
| 1-14 | 0 | 34 | 0 | 1 | 35 | 0 |
| 15 | 16 | 34 | 0.5 | 20 | 35 | 0.6 |
| 16 | 74 | 34 | 2.2 | 71 | 35 | 2.0 |
| 17 | 145 | 34 | 4.3 | 161 | 35 | 4.6 |
| 18 | 175 | 34 | 5.1 | 187 | 35 | 5.3 |
| 19 | 182 | 34 | 5.4 | 196 | 35 | 5.6 |
| 20 | 183 | 34 | 5.4 | 218 | 35 | 6.2 |
| 21 | 190 | 34 | 5.6 | 217 | 35 | 6.2 |
| 22 | 184 | 34 | 5.4 | 209 | 35 | 6.0 |

TABLE III

| | Group 1 | | | Group 2 | | | | | |
|---|---|---|---|---|---|---|---|---|---|
| | (Control) | | | (1 µg 6-MBOA/g of chow) | | | 10 µg 6-MBOA/g of chow | | |
| Week | #eggs/-week | # hens | #eggs/-hen/wk | #eggs/-week | # hens | #eggs/-hen/wk | #eggs/-week | # hens | #eggs/-hen/wk |
| 23 | 178 | 34 | 5.2 | 97 | 18 | 5.4 | 88 | 16 | 5.5 |
| 24 | 159 | 32 | 5.0 | 105 | 18 | 5.8 | 98 | 16 | 6.1 |
| 25 | 173 | 32 | 5.4 | 109 | 18 | 6.1 | 100 | 16 | 6.2 |
| 26 | 190 | 32 | 5.9 | 102 | 18 | 5.7 | 110 | 16 | 6.9 |
| 27 | 186 | 32 | 5.8 | 102 | 18 | 5.7 | 99 | 16 | 6.2 |
| 28 | 172 | 32 | 5.4 | 86 | 18 | 4.8 | 92 | 16 | 5.8 |
| 29 | 165 | 32 | 5.2 | 101 | 18 | 5.6 | 90 | 16 | 5.6 |
| 30 | 160 | 32 | 5.0 | 86 | 18 | 4.8 | 74 | 16 | 4.6 |
| 31 | 196 | 32 | 6.1 | 116 | 18 | 6.4 | 105 | 16 | 6.6 |
| 32 | 163 | 32 | 5.1 | 91 | 18 | 5.1 | 91 | 16 | 5.7 |
| 33 | 171 | 32 | 5.3 | 94 | 18 | 5.2 | 89 | 16 | 5.6 |
| 34 | 167 | 32 | 5.2 | 100 | 18 | 5.6 | 90 | 16 | 5.6 |
| 35 | 153 | 32 | 4.8 | 91 | 18 | 5.1 | 89 | 16 | 5.6 |
| 36 | 140 | 32 | 4.4 | 94 | 18 | 5.2 | 88 | 16 | 5.5 |
| 37 | 139 | 32 | 4.3 | 83 | 17 | 4.9 | 76 | 15 | 5.1 |
| 38 | 131 | 32 | 4.1 | 69 | 17 | 4.1 | 73 | 15 | 4.9 |
| 39 | 139 | 32 | 4.3 | 86 | 17 | 5.1 | 85 | 15 | 5.7 |

NOTE: "wk" represents week.

TABLE IV

| | Group 1 (Control) | Group 2 | |
|---|---|---|---|
| | | 1 μg 6-MBOA/g of chow | 10 μg 6-MBOA/g of chow |
| Week | Percent Fertile Eggs | Percent Fertile Eggs | Percent Fertile Eggs |
| 24 | 71% | 68% | 71% |
| 25 | 67 | 70 | 87 |
| 26 | 76 | 84 | 77 |
| 27 | 76 | 79 | 86 |
| 28 | 76 | 78 | 82 |
| 29 | 75 | 80 | 74 |
| 30 | 70 | 74 | 80 |

Example VII

Four (4) treatments of broiler breeder pullets (approximately 14 weeks of age) and cockerels (approximately 18 weeks of age) were tested for the reproductive effects of 6-MBOA by incorporating varying amounts of 6-MBOA in their feed. The 4 treatments were as follows:

| Treatment | 6-MBOA/ton (907.18 kgs.)of feed |
|---|---|
| 1 | none (control) |
| 2 | 0.5 gm/ton (907.18 kg) |
| 3 | 1.0 gm/ton (907.18 kg) |
| 4 | 5.0 gm/ton (907.18 kg) |

Each treatment consisted of 2 pens wherein each pen housed 44 pullets and 5 cockerels. Water and crushed oyster shells were provided ad libitum. A grower developer ration was fed at a rate of 0.48 lbs. (.218 kg) per bird every other day until the pullets were 20 weeks of age. Then the ration was changed to a breeder hen formula and fed at a rate of 0.27 lbs. (.122 kg) per bird per day. As egg production increased the quantity of breeder hen formula fed to the birds was increased to a maximum of 0.35 lbs. (0.159 kg) per hen per day. Eggs were collected and counted twice daily. Table V lists egg production of the 4 treatments in average percent egg production based on one egg per hen per day. The term "average percent egg production" is a term which means the average percentage of hens in a treatment which lay one egg per day. Thus, if the "average percent egg production" for a treatment of 20 hens is 100, this

indicates that on the average 20 eggs in total are produced per day from the group of 20 hens in that treatment. If the "average percent egg production" for a treatment of 20 hens is 50, this indicates that on the average 10 eggs in total are produced per day from the group of 20 hens in that treatment.

TABLE V

| Week | Control | Treatment: 0.5 gm 6-MBOA /ton* of feed) | 1.0 gm 6-MBOA /ton* of feed) | 5.0 gm 6-MBOA /ton* of feed) |
|---|---|---|---|---|
| 1 | 1.33 | .16 | .99 | 0 |
| 2 | 3.68 | 2.99 | 4.98 | 2.52 |
| 3 | 13.55 | 14.12 | 14.45 | 14.12 |
| 4 | 36.54 | 32.39 | 36.88 | 39.12 |
| 5 | 51.70 | 54.82 | 53.33 | 53.06 |
| 6 | 60.16 | 69.60 | 65.60 | 69.79 |
| 7 | 71.40 | 82.35 | 73.20 | 73.53 |
| 8 | 74.50 | 82.48 | 71.67 | 78.27 |
| 9 | 73.64 | 85.83 | 74.94 | 78.58 |
| 10 | 71.35 | 77.35 | 75.67 | 78.31 |
| 11 | 71.69 | 71.43 | 71.85 | 74.54 |
| 12 | 69.99 | 71.09 | 69.81 | 73.87 |
| 13 | 70.05 | 65.46 | 67.73 | 70.05 |
| 14 | 64.46 | 73.97 | 64.98 | 67.14 |
| 15 | 68.93 | 73.99 | 69.57 | 68.71 |
| 16 | 62.32 | 73.27 | 66.83 | 67.63 |
| 17 | 61.58 | 74.90 | 63.45 | 70.43 |
| 18 | 61.02 | 69.77 | 62.92 | 69.75 |
| 19 | 59.39 | 68.19 | 60.97 | 66.12 |
| 20 | 58.15 | 70.72 | 58.80 | 64.72 |
| 21 | 58.32 | 66.70 | 57.10 | 62.17 |
| 22 | 49.84 | 60.30 | 57.49 | 60.08 |
| 23 | 55.47 | 64.81 | 55.74 | 56.64 |
| 24 | 56.48 | 64.29 | 60.66 | 59.56 |
| 25 | 59.34 | 64.46 | 65.47 | 62.79 |
| 26 | 58.46 | 62.37 | 64.18 | 58.04 |
| 27 | 61.00 | 63.59 | 63.68 | 61.97 |
| 28 | 62.78 | 66.38 | 65.73 | 65.07 |
| 29 | 63.88 | 65.86 | 63.44 | 66.46 |
| 30 | 62.96 | 66.55 | 63.26 | 63.18 |
| 31 | 60.90 | 66.03 | 63.14 | 65.91 |
| 32 | 58.79 | 63.24 | 59.79 | 61.96 |
| 33 | 60.59 | 62.37 | 57.12 | 61.80 |
| 34 | 57.87 | 64.40 | 60.87 | 63.71 |
| 35 | 61.01 | 64.76 | 64.94 | 63.57 |
| 36 | 55.27 | 62.03 | 61.64 | 62.37 |

*ton = 907.18 kgs

Starting Materials

The compounds employed in the practice of this invention can, in general, be prepared employing procedures taught in one or more of the following references: U.S. Patent 3,812,138; Agric. Biol. Chem., 1975, 39(3), 683-5; J. Chem. Soc. Perkin Trans.1, 1975, (20), pp. 2022-5 (CA 84:43489 t); J. Pharm. Soc. Japan, 76, pp. 1002-5, (1956), (CA 51:2738 efg); Indian J. Chem, 1974, 12(3), pp. 227-35, (CA 81:152199 g); German Offer. 2,131,366 (CA 78:124576 x, U.S. Patent 3,812,138) and Mol. Pharmacal, 1972, 8(4), pp. 398-409 (CA 77:71863 j).

Claims

1. A method of stimulating reproductive performance in mammalian and avian species which comprises administering to said mammalian and avian species an effective reproductive stimulating amount of one or more active compounds of Formula I, II or III:

(I) a compound of the formula:

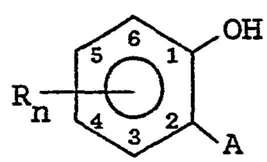

wherein

R represents $C_1$-$C_4$ alkoxy, with the proviso that R is in the 4 or 5 ring position,

n represents one of the integers 0, 1 or 2, and

A represents -OH, $-NH_2$ or $NH\overset{O}{\overset{\|}{C}}R'$ where R' represents $C_1$-$C_4$ alkyl,

or physiologically acceptable salts thereof;

(II) a compound of the formula:

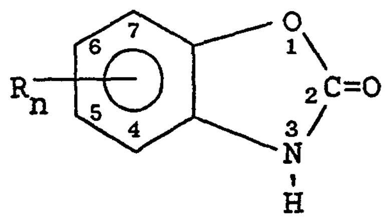

wherein

R represents $C_1$-$C_4$ alkoxy, with the proviso that R is in the 5 or 6 ring position, and

n represents one of the integers 0, 1 or 2; and

(III) a compound of the formula:

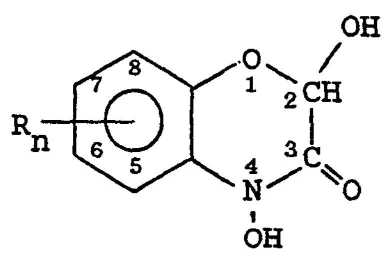

wherein

R represents $C_1$-$C_4$ alkoxy, with the proviso that R is in the 6 or 7 ring position, and

n represents one of the integers 0, 1 or 2;

or physiologically acceptable salts thereof.

2. The method of Claim 1 wherein said active compound is administered to said mammalian and avian species in an amount in the range of from 0.01 to 2 milligrams per kilogram of bodyweight per day.

3. The method of Claim 1 wherein said active compound is synthetically produced 6-methoxy-2-benzoxazolinone.

4. The method of Claim 1 wherein said active compound is synthetically produced, substantially pure 6-methoxy-2-benzoxazolinone.

5. The method of Claim 1 wherein said active compound is 2-amino-5-methoxyphenol, 6-methoxy-2-benzoxazolinone, 2,4-dihydroxy-7-methoxy-1, 4-(2H)-benzoxazin-3-one, 2-hydroxy-4-methoxyacetanilide, 2-hydroxy-4-ethoxyacetanilide, 5-methoxy-2-benzoxazolinone, 2-hydroxy-5-methoxyacetanilide, or a physiologically-acceptable salt or a mixture thereof.

6. An animal feed comprising from 0.00001% to 0.0052% by weight of one or more reproductive stimulating active compounds of Formula I, II or III

(I) a compound of the formula:

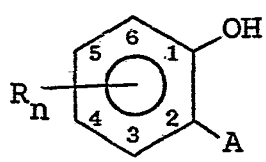

wherein

R represents $C_1$-$C_4$ alkoxy, with the proviso that R is in the 4 or 5 ring position,

n represents one of the integers 0, 1 or 2, and

A represents -OH, $-NH_2$ or $NH\overset{O}{\overset{\|}{C}}R'$ where R' represents $C_1$-$C_4$ alkyl,

or physiologically acceptable salts thereof;

(II) a compound of the formula:

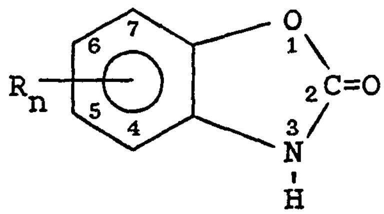

wherein

R represents $C_1$-$C_4$ alkoxy, with the proviso that R is in the 5 or 6 ring position, and

n represents one of the integers 0, 1 or 2;

and

(III) a compound of the formula:

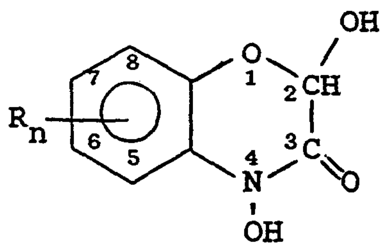

wherein

R represents $C_1$-$C_4$ alkoxy, with the proviso that R is in the 6 or 7 ring position, and

n represents one of the integers 0, 1 or 2;

or physiologically acceptable salts thereof.

7. The animal feed of Claim 6 wherein said active compound is synthetically produced, 6-methoxy-2-benzoxazolinone.

8. The animal feed of Claim 6 wherein said active compound is synthetically produced, substantially pure 6-methoxy-2-benzoxazolinone.

9. The animal feed of Claim 6 wherein the active compound is 2-amino-5-methoxyphenol, 6-methoxy--2-benzoxazolinone, 2,4-dihydroxy-7-methoxy-1,4-(2H)--benzoxazin-3-one, 2-hydroxy-4-methoxyacetanilide, 2-hydroxy-4-ethoxyacetanilide, 5-methoxy-2-benzoxazolinone, 2-hydroxy-5-methoxyacetanilide, or a physiologically -acceptable salt or a mixture thereof.

10. An animal feed concentrate comprising from 0.001 to 99 percent by weight of one or more reproductive stimulating active compounds of Formula I, II or III:

(I) a compound of the formula:

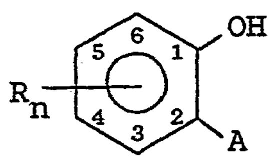

wherein

R represents $C_1$-$C_4$ alkoxy, with the proviso that R is in the 4 or 5 ring position,

n represents one of the integers 0, 1 or 2, and

A represents -OH, $-NH_2$ or $NH\overset{O}{\overset{\|}{C}}R'$ where R' represents $C_1$-$C_4$ alkyl,

or physiologically acceptable salts thereof;

(II) a compound of the formula:

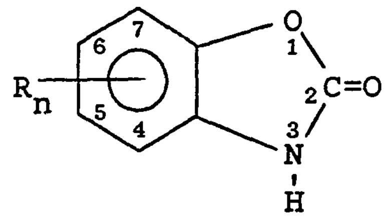

wherein

R represents $C_1$-$C_4$ alkoxy, with the proviso that R is in the 5 or 6 ring position, and

n represents one of the integers 0, 1 or 2;

and

(III) a compound of the formula:

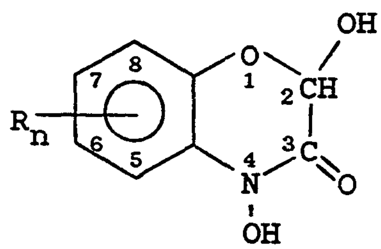

wherein

R represents $C_1$-$C_4$ alkoxy, with the proviso that R is in the 6 or 7 ring position, and

n represents one of the integers 0, 1 or 2;

or physiologically acceptable salts thereof.

0070016

European Patent Office

# EUROPEAN SEARCH REPORT

Application number

EP 82 10 6177.7

| DOCUMENTS CONSIDERED TO BE RELEVANT | | | CLASSIFICATION OF THE APPLICATION (Int. Cl. 3) |
|---|---|---|---|
| Category | Citation of document with indication, where appropriate, of relevant passages | Relevant to claim | |
| | No documents considered to be relevant | | A 23 K 1/16 |
| | | | TECHNICAL FIELDS SEARCHED (Int.Cl. 3) |
| | | | A 23 K 1/00 |
| | | | CATEGORY OF CITED DOCUMENTS<br>X: particularly relevant if taken alone<br>Y: particularly relevant if combined with another document of the same category<br>A: technological background<br>O: non-written disclosure<br>P: intermediate document<br>T: theory or principle underlying the invention<br>E: earlier patent document, but published on, or after the filing date<br>D: document cited in the application<br>L: document cited for other reasons |
| X | The present search report has been drawn up for all claims | | &: member of the same patent family, corresponding document |

| Place of search | Date of completion of the search | Examiner |
|---|---|---|
| Berlin | 26-08-1982 | SCHULTZE |

EPO Form 1503.1 06.78